(19) Europäisches Patentamt
European Patent Office
Office européen des brevets

(11) Veröffentlichungsnummer: **0 353 448 B1**

(12) **EUROPÄISCHE PATENTSCHRIFT**

(45) Veröffentlichungstag der Patentschrift: **27.10.93**

(51) Int. Cl.$^5$: **C07C 323/52**, C07C 303/40, A61K 31/18, C07C 311/16

(21) Anmeldenummer: **89111421.7**

(22) Anmeldetag: **23.06.89**

# (54) Sulfonamide, Verfahren zu ihrer Herstellung sowie diese Verbindungen enthaltende Arzneimittel.

(30) Priorität: **25.06.88 DE 3821540**

(43) Veröffentlichungstag der Anmeldung:
**07.02.90 Patentblatt 90/06**

(45) Bekanntmachung des Hinweises auf die Patenterteilung:
**27.10.93 Patentblatt 93/43**

(84) Benannte Vertragsstaaten:
**AT BE CH DE ES FR GB GR IT LI LU NL SE**

(56) Entgegenhaltungen:
**EP-A- 0 000 826**
**EP-A- 0 223 593**
**EP-A- 0 255 728**
**US-A- 4 752 613**

(73) Patentinhaber: **BOEHRINGER MANNHEIM GMBH**

**D-68298 Mannheim(DE)**

(72) Erfinder: **Witte, Ernst-Christian, Dr.rer.nat.**
**Beethovenstrasse 2**
**D-6800 Mannheim 1(DE)**
Erfinder: **Stegmeier, Karlheinz, Dr.rer.nat.**
**Kirchbergstrasse 17**
**D-6148 Heppenheim(DE)**
Erfinder: **Doerge, Liesel, Dr.med.**
**Schwalbenstrasse 26**
**D-6840 Lampertheim(DE)**
Erfinder: **Slater, Robert Antony, Dr.**
**65 Longmead**
**Letchworth**
**Hertfordshire SG6 4HR(GB)**

**EP 0 353 448 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Anmeldung betrifft substituierte Sulfonamide und ihre Derivate, Verfahren zu ihrer Herstellung sowie Arzneimittel, die solche Verbindungen der nachfolgenden Formel I enthalten:

$$R^1-SO_2-N-A-Q-B-Y \qquad (I)$$
$$|$$
$$R^2$$

In Formel I bedeuten

- $R^1$ eine niedere Alkyl- oder Alkenylgruppe mit 1-6 C-Atomen einen Cycloalkylrest mit 3-7 C-Atomen, einen Aralkyl-, Aralkenyl- oder Arylrest, wobei der jeweilige Arylrest ein- oder mehrfach durch Halogen, $C_1$-$C_6$ Alkyl, $C_1$-$C_6$-Alkoxy, Hydroxyl, Trifluormethyl, Cyan, Nitro, Amino, $C_1$-$C_6$ Alkylamino, $C_2$-$C_{12}$-Dialkylamino, $C_1$-$C_6$-Acylamino, $C_1$-$C_{16}$-Acyl, $C_1$-$C_6$-Alkylsulfenyl, -sulfinyl und -sulfonyl oder durch Azid substituiert sein kann;
- $R_2$ ein Wasserstoffatom, eine $C_1$-$C_6$-Alkyl-, eine Aralkyl-, eine Aralkenyl- oder eine Acylgruppe,
- A und B eine gesättigte oder ungesättigte Alkylenkette mit 1-10 Kohlensstoffatomen, die einoder mehrfach durch $C_1$-$C_3$ Alkylgruppen substituiert sein kann, wobei die Summe der Kohlenstoffatome der Ketten A plus B wenigstens 4, maximal jedoch 11 beträgt,
- Q Sauerstoff, Schwefel, eine Sulfonyl- oder eine Sulfinylgruppe, oder eine Aminogruppe

$$-N-,$$
$$|$$
$$R^2$$

  wobei für $R^2$ die obige Defination gilt,
  und
- Y eine freie Carbonsäuregruppe, ein Carbonsäureester, ein Carbonsäureamid, oder Hydroxymethyl oder Tetrazolyl.

Benzolsulfonamide, die am Stickstoffatom eine durch Schwefel unterbrochene Alkylkette tragen, sind in EP-826 beschrieben, jedoch trägt das endständige C-Atom eine Aminogruppe. Ferner wirken diese Verbindungen gegen Fibrinthrombosen.

In EP-A-233 593, EP-A-255 728 und US-A-4,849,563 sind Benzolsulfonamide mit $TXA_2$-antagonistischer Wirkung beschrieben, die am Stickstoffatom eine Alkylkette tragen, die durch cyclische Gruppen unterbrochen ist.

Die neuen Verbindungen der allgemeinen Formel I weisen wertvolle pharmakologische Eigenschaften auf, insbesondere zeigen sie eine ausgezeichnete antagonistische Wirkung gegenüber Thromboxan $A_2$ sowie gegen Prostaglandin-endoperoxide. Sie inhibieren die Aggregation von Blutplättchen und verhindern die Konstriktion der glatten Muskulatur sowie die Bronchokonstriktion. Sie sind außerdem wertvolle Heilmittel zur Behandlung pathologischer Veränderungen der Nierenfunktion.

Diese Eigenschaften machen sie zu wertvollen Heilmitteln zur Behandlung beispielweise von cardiovaskulären Erkrankungen und von Asthma und zur Prophylaxe einer Schocklunge. Sie können weiterhin verwendet werden bei Organtransplantationen und bei der Nierendialyse und sind geeignet, Rezidive bei Magengeschwüren zu verhindern. Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können beispielsweise gegen cerebrale und ischaemische Zustände eingesetzt werden.

A und B sind jeweils acyclische, gesättigte oder ungesättigte Kohlenwasserstoffgruppen mit 1-10 linearen Kohlenstoffatomen, von denen eines oder mehrere an beliebiger Stelle durch eine oder zwei $C_1$-$C_3$-Alkylgruppen substituiert sein können, die auch gemeinsam eine Alkylenkette $-(CH_2)_m-$ bilden können, wobei m die Bedeutung 2-5 hat und die Summe der Ketten-Kohlenstoffatome der Ketten A plus B

mindestens 4, maximal jedoch 11 beträgt.

Bevorzugte Kohlenwasserstoffgruppen A sind die folgenden:

$-(CH_2)_n-$ wobei n die Zahlen 1 bis 6 darstellt, sowie

$-(CH_2)_m-CH=CH-(CH_2)_n-$ mit der Bedeutung
m = 1 und 2 und
n = 2,3 4 und 5.

Die bevorzugten Kohlenwasserstoffgruppen B sind die folgenden:

$-(CH_2)_p-$ wobei p die Zahlen 1 bis 7 darstellt, sowie

$-(CH_2)_q-C(CH_3)_2-$ mit der Bedeutung q = 0 bis 6 und

$-(CH_2)_r-C(CH_3)_2-CH_2-$ wobei r die Zahlen 0 bis 5 darstellt.

Bevorzugte Substituenten der A- bzw. B-Ketten sind Methyl-und Ethyl, insbesondere Methyl. Sollten die beiden Substituenten der A- bzw. B-Ketten gemeinsam eine Alkylengruppe $-(CH_2)_m-$ bedeuten, so ist m = 5 bevorzugt, und es liegt ein 6-Ring zugrunde, wenn die Substituenten am gleichen C-Atom stehen.

Niedere Alkyl- oder Alkenylreste $R_1$ sind vorzugsweise Methyl, Ethyl, Propyl sowie Propenyl. Cycloalkylreste $R_1$ sind vorzugsweise Cyclopentyl, Cyclohexyl oder Cycloheptyl.

Ein Aralkylrest $R_1$ bedeutet bevorzugt Benzyl oder Phenethyl, ein Aralkenylrest $R_1$ Cinnamyl und ein Arylrest $R_1$ Phenyl oder Naphthyl, wobei die jeweiligen Phenylreste ein- oder mehrfach durch Halogen, Methyl, Methoxy, Trifluormethyl, Cyan, Nitro, Amino, Hydroxy, Methylamino, Diethylamino, Acetamino, Acetyl, Octanoyl, Hexadecanoyl, Methylsulfenyl, Methylsulfinyl, Methylsulfonyl oder Azid substituiert sein können. Bevorzugte Reste $R_1$ sind 4-Chlorphenyl-, 4-Bromphenyl-, 4-Fluorphenyl, 3-Trifluormethylphenyl, 4-Methylphenyl,4-Cyanophenyl, 4-Methoxyphenyl oder 3-Methylphenyl, insbesondere 4-Chlorphenyl, 4-Methoxyphenyl oder 3-Trifluormethylphenyl.

$R_2$ bedeutet normalerweise Wasserstoff, kann aber auch Methyl, Benzyl, 4-Chlorbenzyl, Cinnamyl, 4-Chlorcinnamyl Benzoyl oder Acetyl bedeuten.

Als Halogenatom kommen Fluor, Chlor oder Brom in Frage.

Beansprucht werden auch alle durch Verzweigung der A- bzw. B-Kette entstehenden optischen Isomere, Isomerengemische bzw. Razemate. Beansprucht werden weiterhin für den Fall, daß die Ketten A bzw. B Alkencharakter tragen, sämtliche sich daraus ergebende mögliche Isomere, d.h. die (Z)-, die (E)-Form und Gemische aus beiden.

Die Herstellung der Verbindungen der allgemeinen Formel I erfolgt für den Fall, daß Q ein Sauerstoff-, ein Schwefelatom oder eine Gruppe $-NR^2-$ darstellt, in der Weise, daß man

a) eine Verbindung der allgemeinen Formel II

Z-O-A-X (II),

in welcher A die oben angegebene Bedeutung hat, Z eine Schutzgruppe für die Hydroxylfunktion ist und X hier und in allen anderen Fällen einen reaktiven Rest (wie z.B. Halogen oder einen Sulfonsäureesterrest wie z.B. Mesylat oder Tosylat) darstellt,
in an sich bekannter Weise mit einer Verbindung der allgemeinen Formel III

H-Q-B-Y (III),

in welcher Q Sauerstoff, Schwefel oder eine Funktion $-NR^2-$ darstellt und B und Y die oben angegebene Bedeutung haben, umsetzt.
Anschließend spaltet man die Schutzgruppe Z ab, wandelt die Hydroxylgruppe der nun entstandenen Verbindung IV

HO-A-Q-B-Y (IV)

in eine Gruppe X um und setzt die Verbindung der allgemeinen Formel V

X-A-Q-B-Y (V)

mit einem Sulfonamid der allgemeinen Formel VI

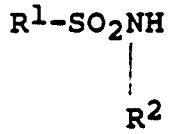

(VI)

um.

Die in den Formeln V und VI benutzten Symbole X, A, Q, B, Y, $R^1$ und $R^2$ haben jeweils die oben angegebene Bedeutung.

b) Umgekehrt lassen sich Verbindungen der allgemeinen Formel I auch darstellen, indem man eine Verbindung der allgemeinen Formel VII

Z-O-A-Q-H (VII)

in welcher Z und A die oben angegebene Bedeutung haben und Q Sauerstoff, Schwefel oder eine - $NR^2$ - Gruppe darstellen,

mit einer Verbindung der allgemeinen Formel VIII

X-B-Y (VIII)

in welcher X, B und Y die oben angegebene Bedeutung haben, hier aber zusätzlich eine Diazogruppe darstellen kann, zur Umsetzung bringt, wiederum die Schutzgruppe Z abspaltet, die Verbindung IV in V umwandelt und letztere mit einem Sulfonamid VI reagieren läßt.

Für den Fall, daß Q ein Schwefelatom darstellt, ist es auch möglich, statt der geschützten Verbindung VII eine Verbindung VIIa

HO-A-Q-H (VIIa)

d.h. eine Verbindung VII ohne geschützte Hydroxylgruppe, mit einer Verbindung VIII reagieren zu lassen, wobei man unmittelbar IV erhält, welches sinngemäß weiter umgesetzt wird.

c) Ein weiteres Verfahren besteht in der Umsetzung eines Sulfonamides VI mit einer Verbindung der allgemeinen Formel IX

$X^1$-A-$X^2$ (IX),

in welcher $X_1$ und $X_2$ zwei gleiche oder verschiedene reaktive Reste im Sinne der vorherigen Definition für X darstellen, zu einer Verbindung der allgemeinen Formel X

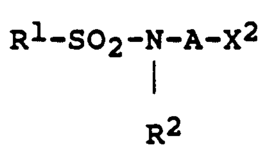

(X).

Durch Umwandeln der Gruppe $X^2$ in die Gruppe -QH erhält man dann Verbindungen der allgemeinen Formel XI

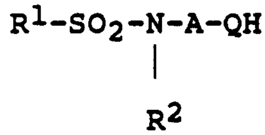

(XI),

welche durch chemische Reaktion mit Verbindungen VIII in die gewünschten Produkte der allgemeinen Formel I überführt werden.

d) Für den Fall, daß Q der Formel I eine $>$NH-Gruppe darstellt,
läßt man eine Verbindung der allgemeinen Formel XII

$Z\text{-}O\text{-}A^1\text{-}CH=O$ (XII)

mit einer Verbindung der allgemeinen Formel XIII

$H_2N\text{-}B\text{-}Y$ (XIII)

reagieren, oder aber man bringt eine Verbindung der allgemeinen Formel XIV

$Z\text{-}O\text{-}A\text{-}NH_2$ (XIV)

mit einem Aldehyd der allgemeinen Formel XV

$O=CH\text{-}B^1\text{-}Y$ (XV)

zur Umsetzung. Die in beiden Fällen entstehenden SCHIFF-Basen werden zum Amin der allgemeinen Formel XVI

Z-O-A-NH-B-Y (XVI)

hydriert. In den Formeln XII und XV bedeuten $A^1$ und $B^1$ jeweils um ein Kohlenstoffatom verkürzte Reste A und B: Dieses Kohlenstoffatom trägt hier den Oxo-Sauerstoff.
Da nach Abspaltung von Z und Umwandlung der Hydroxylgruppe der Verbindung XVI in eine Gruppe X diese Gruppe X mit der sekundären Aminogruppe reagieren kann, muß diese Aminogruppe intermediär geschützt werden.
Bevorzugt ist ein Verfahren, bei dem man
d1) einen Aldehyd der allgemeinen Formel XVII

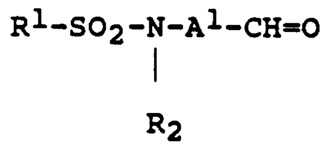

(XVII)

mit einem Amin der allgemeinen Formel XIII bzw. ein Amin der allgemeinen Formel XVIII

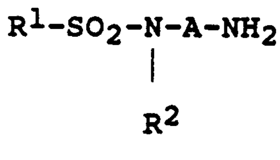

(XVIII)

mit einem Aldehyd der allgemeinen Formel XV umsetzt und die jeweils entstehenden SCHIFF-Basen hydriert.
e) Verbindungen der allgemeinen Formel I, in denen Q eine Sulfinylgruppe darstellt, werden durch Umsetzung der entsprechenden Sulfenylverbindungen mit geeigneten Oxidationsmitteln dargestellt.
e1) Die Herstellung von Verbindungen der allgemeinen Formel I, in denen Q eine Sulfonylgruppe bedeutet, erfolgt entweder

- durch Oxidation der entsprechenden Sulfenylverbindungen

oder

- durch Weiteroxidation der entsprechenden Sulfinylverbindungen

mit geeigneten Oxidationsmitteln.

Die Umsetzung der Verbindungen der allgemeinen Formel III mit Verbindungen der allgemeinen Formel II bzw. die Umsetzung von Verbindungen VII mit Verbindungen VIII erfolgt zweckmäßig unter Zusatz eines säurebindenden Mittels. Als solche Agenzien kommen z.B. Alkalicarbonate, Alkalihydroxide, aber auch Alkoholate niederer aliphatischer Alkohole infrage. Als Reaktionsmedium kommen niedere aliphatische Alkohole, aliphatische Ketone oder aprotische Lösungsmitteln wie DMF oder Benzol infrage. Will man eine Verbindung VII, in deren allgemeinen Formel Q ein Sauerstoffatom darstellt, mit VIII umsetzen, so benutzt man zweckmäßig solche Verbindungen VIII, in denen allg. Formel X eine Diazogruppe darstellt. Die Kondensation erfolgt in Gegenwart von Kupferpulver z.B. in Essigester.

Die Abspaltung der Schutzgruppe Z erfolgt nach literaturbekannten Verfahren. So wird z.B. ein Tetrahydropyranylether durch Behandeln mit warmen, verdünnten Mineralsäuren gespalten.

Die Umwandlung der freien Hydroxylgruppe der Verbindungen IV in eine reaktive Gruppe X erfolgt z.B. durch Umsetzen mit Mesylchlorid oder Tosylchlorid in Pyridin, wobei Mesylate oder Tosylate entstehen. Als reaktive Gruppe X kommen aber auch Halogenatome infrage.

Zur Umwandlung von aliphatischen Hydroxyl in Halogen stehen zahlreiche Verfahren zur Verfügung. Bevorzugt ist die Umwandlung in Brom, welche entweder durch Umsetzen der Hydroxyverbindung IV mit einem anorganischen Säurebromid wie z.B. $PBr_3$ erfolgt, oder man bildet zunächst ein Mesylat oder ein Tosylat und läßt dieses mit Lithiumbromid in Aceton reagieren, wobei die Bromide in hoher Ausbeute entstehen.

Die Reaktion zwischen einer Verbindung der allgemeinen Formel V mit einem Sulfonamid der Formel VI wird bevorzugt so durchgeführt, daß man im Falle primärer Sulfonamide zwei Mol Sulfonamid mit einem Mol Natriumalkoholat in Alkohol umsetzt, das Gemisch eindampft und das trockne Natriumsalz mit V in DMF reagieren läßt. Im Falle sekundärer Sulfonamide arbeitet man dagegen mit einem Molverhaltnis von 1:1:1. Wählt man zur Herstellung von Verbindungen der allgemeinen Formeln XVI bzw. I (Q = $>$NH) den Weg über die Bildung von SCHIFF-Basen, so läßt man zweckmäßig den entsprechenden Aldehyd mit dem notwendigen primären Amin in einem geeigneten Lösungsmittel wie z.B. Ethanol reagieren und reduziert die SCHIFF-Base, ohne sie zu isolieren, durch Zugabe von Natriumborhydrid. Im Falle wenig aktiver Reaktanten wird die Bildung der SCHIFF-base durch azeotropes Auskreisen des gebildeten Wassers erzwungen. Die Reduktion der SCHIFF-basen kann auch durch Hydrieren an einem Katalysator wie z.B. Raney-Nickel oder Palladium-auf-Kohle erfolgen. Die Oxidation der Sulfenylverbindungen zu den Sulfinyl- bzw. Sulfonylverbindungen erfolgt entweder mit einem anorganischen Oxidationsmittel wie $H_2O_2$, $NaJO_4$, $KMnO_4$, NaClO oder Oxon, oder mit einer organischen Persäure wie z.B. m-Chlorperbenzoesäure. Als Lösungsmittel kommen Wasser, niedere aliphatische Carbonsäuren wie z.B. Essigsäure, oder chlorierte Kohlenwasserstoffe in Betracht.

Die Herstellung der Sulfinylverbindungen erfolgt durch Zugabe von einem Äquivalent des betreffenden Oxidationsmittels zur Sulfenylverbindung.

Zur Vermeidung der Bildung der Sulfonylverbindungen kann auch mit Sauerstoff in Gegenwart von Cer-ammon-nitrat in Acetonitril, unter Bedingungen wie sie von Riley et al. J. Chem. Soc. Chem. Comm. 1986, 1097 angegeben werden, oxidiert werden.

Vorteilhaft ist auch die Oxidation mittels Oxon in Methylenchlorid in Gegenwart eines Phasentransfer-Katalysators in Analogie zu Evans et al. Synth. Comm. 16, 1207 (1986).

Die Herstellung der Sulfonylverbindungen erfolgt durch Zugabe von zwei Äquivalenten des Oxidationsmittels zur Sulfenylverbindung oder von einem Äquivalent zur Sulfinylverbindung.

Die nachträgliche Einführung von Alkylgruppen $R^2$ in einen sekundären Sulfonamidrest erfolgt durch Umsetzen des Natriumsalzes des Sulfonamids mit einem Alkylhalogenid oder einem Alkylmesylat bzw. -tosylat in einem polaren Lösungsmittel wie z.B. DMF.

Die nachträgliche N-Alkylierung einer Verbindung der allgemeinen Formel I, in welcher Q = -NH- bedeutet, kann nach bekannten Methoden durchgeführt werden. Bevorzugt ist die Umsetzung mit einem Alkylhalogenid oder einem Dialkylsulfat in Gegenwart eines säurebindenden Mittels wie z.B. Natriumhydroxid.

Zur Acylierung des Sulfonamidrestes setzt man das sekundäre Sulfonamid I ($R^2$ = H) mit einem Acylhalogenid um, wobei man als Halogenwasserstoff-akzeptor ein tertiäres Amin wie Triethylamin oder Pyridin verwendet. Als Lösungsmittel benutzt man entweder einen Überschuß des tertiären Amins oder ein inertes Lösungsmittel wie z.B Methylenchlorid, Benzol oder DMF. In gleicher Weise erfolgt die nachträgliche Einführung von Acylresten $R^2$ in den sekundären Aminrest (I, Q = -NH-). Anstelle des Acylhalogenids kann in beiden Fällen auch ein gemischtes Anhydrid oder ein aktiver Ester als Acylierungsmittel dienen.

Die gegebenenfalls im Anschluß an die Kondensation durchzuführende Umwandlung des Substituenten Y erfolgt beispielsweise durch Verseifen der Carbonsäureester ($R^4$ = Alkyl) zu den entsprechenden Carbonsäuren ($R^4$ = H) mit Mineralsäuren oder Alkalihydroxiden in einem polaren Lösungsmittel (z.B. Wasser, Methanol, Ethanol, Dioxan oder Aceton). Vorteilhaft wird die Verseifung mit einer starken Base (wie Natrium- oder Kaliumhydroxid) in einem Gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest $R^4$ durch Umestern in einen Ester mit einem anderen Rest $R^4$ umwandeln. Die Veresterung der Carbonsäuren wird zweckmäßig in Gegenwart eines sauren Katalysators, wie z.B. Chlorwasserstoff, Schwefelsäure, p-Toluolsulfonsäure oder in Gegenwart eines stark sauren Ionenaustauschharzes vorgenommen. Umesterungen hingegen erfordern den Zusatz einer geringen Menge einer basischen Substanz, z.B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol oder Alkohole mit anderen funktionellen Gruppen, wie Ethanolamin oder Glykolether.

Die erfindungsgemäßen, von den Carbonsäuren der allgemeinen Formel I abgeleiteten Amide werden bevorzugt nach an sich bekannten Methoden aus den Carbonsäuren oder ihren reaktiven Derivaten (wie z.B. Carbonsäurehalogeniden, -estern, -aziden, -anhydriden oder gemischten Anhydriden) durch Umsetzung mit Aminen hergestellt. Als Aminokomponenten kommen z.B. Ammoniak, Alkylamine, Dialkylamine, aber auch Aminoalkohole wie z.B. Ethanolamin und 2-Aminopropanol sowie Aminosäuren wie z.B. p-Aminobenzoesäure, $\beta$-Alanin und andere infrage. Andere wertvolle Aminkomponenten sind Alkyl-, Aralkyl- und Arylpiperazine, sowie 5-Aminotetrazol.

Zur Herstellung von Verbindungen der allgemeinen Formel I, in denen Y die Bedeutung $-CH_2OH$ hat, geht man zweckmäßig von Verbindungen I aus, in denen Y eine Carboxylfunktion ($R^4$ = H) oder eine Esterfunktion ($R^4$ = Alkyl) darstellt.

Zur Reduktion der Carboxylfunktion sind alle für diesen Zweck üblichen Reduktionsmittel geeignet, z.B. komplexe Hydride wie Lithiumaluminium-hydrid oder Dibal, oder Boran-addukte wie z.B. $BH_3$ . THF. Die Reduktion kann aber auch vorteilhaft durch Reduzieren eines Derivates der Carbonsäure, z.B. eines gemischten Anhydrids aus der Carbonsäure und einem Kohlensäurehalbester, erfolgen. Als Reduktionsmittel verwendet man hier bevorzugt komplexe Borhydride wie z.B. $NaBH_4$ in protischen Lösungsmitteln.

Für die Reduktion von Carbonsäureestern zu primären Alkoholen gibt es zahlreiche literaturbekannte Verfahren. Bevorzugte Reduktionsmittel sind auch hier komplexe Aluminiumhydride wie z.B. Lithiumalanat oder DIBAL.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg Tag in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Estern und Amiden die folgenden:

1) 8-(4-Chlor-benzolsulfonamido)-2.2-dimethyl-4-thia-octansäure
2) 8-(4-Chlor-benzolsulfonamido)-2.2-dimethyl-5-thia-octansäure
3) 8-(4-Chlor-benzolsulfonamido)-2.2-dimethyl-7-thia-octansäure
4) 8-(4-Chlor-benzolsulfonamido)-3.3-dimethyl-4-thia-octansäure
5) 8-(4-Chlor-benzolsulfonamido)-3.3-dimethyl-5-thia-octansäure
6) 8-(4-Chlor-benzolsulfonamido)-3.3-dimethyl-7-thia-octansäure
7) (Z)-8-(4-Chlor-benzolsulfonamido)-3-thia-6-en-octansäure
8) (E)-8-(4-Chlor-benzolsulfonamido)-3-thia-6-en-octansäure
9) 8-(4-Chlor-benzolsulfonamido)-3-aza-octansäure
10) N-(4-Chlorbenzyl)-8-(4-chlor-benzolsulfonamido)-3-aza-octansäure
11) 8-(4-Chlor-benzolsulfonamido)-3-oxa-octansäure
12) 8-(4-Fluor-benzolsulfonamido)-2.2-dimethyl-4-thia-octansäure
13) 8-(4-Methyl-benzolsulfonamido)-3.3-dimethyl-5-thia-octansäure
14) 9-(4-Chlor-benzolsulfonamido)-2.2-dimethyl-4-thia-nonan-säure
15) 9-(4-Chlor-benzolsulfonamido)-2.2-dimethyl-5-thia-nonansäure
16) 9-(4-Chlor-benzolsulfonamido)-2.2-dimethyl-7-thia-nonansäure
17) 9-(4-Chlor-benzolsulfonamido)-2.2-dimethyl-8-thia-nonansäure
18) 9-(4-Chlor-benzolsulfonamido)-3.3-dimethyl-5-thia-nonansäure
19) 9-(4-Chlor-benzolsulfonamido)-3.3-dimethyl-7-thia-nonansäure
20) 9-(4-Chlor-benzolsulfonamido)-3.3-dimethyl-8-thia-nonansäure
21) (Z)-9-(4-Chlor-benzolsulfonamido)-3-thia-6-en-nonansäure
22) 9-(4-Chlor-benzolsulfonamido)-3-aza-nonansäure
23) N-Methyl-9-(4-chlor-benzolsulfonamido)-3-aza-nonansäure
24) 9-(3-Methyl-benzolsulfonamido)-3-thia-nonansäure
25) 9-(4-Fluor-benzolsulfonamido)-3.3-dimethyl-6-thia-nonansäure
26) 8-(4-Chlor-benzolsulfonamido)-3.3-dimethyl-6-aza-octansäure
27) 9-(4-Chlor-benzolsulfonamido)-3.3-dimethyl-6-aza-nonansäure

## Beispiel 1

### 8-(4-Chlor-benzolsulfonamido)-3-thia-octansäure

a) 8-Hydroxy-3-thia-octansäure-ethylester
Zu einem Gemisch aus 20.4 g (0.17 mol) Thioglykolsäure-ethylester, 200 ml Ethanol und 0.17 mol Natriummethylat (in Form einer 30proz. Lösung) tropft man eine Lösung aus 28.4 g (0.17 mol) 5-Brom-pentanol und 200 ml Ethanol,
erhitzt zwei Stdn. auf Rückflußtemperatur und destilliert anschließend im Vakuum das Ethanol ab. Der Rückstand wird in Ether aufgenommen, die Lösung wird mehrmals mit Eiswasser extrahiert, dann über Natriumsulfat getrocknet und eingedampft.
Ausbeute 31.9 g (91 % d.Th.), farbloses Öl.
b) 8-(Methansulfonyloxy)-3-thia-octansäure-ethylester
Bei -5°C tropft man innerhalb 30 min zu einem Gemisch aus 30.1 g (0.146 mol) 8-Hydroxy-3-thia-octansäure-ethylester, 200 ml Methylenchlorid und 22.2 g (0.219 mol) abs. Triethylamin eine Lösung aus 16.7 g (0.146 mol) Methansulfonylchlorid und 150 ml Methylenchlorid und rührt weitere 30 min bei 0°C. Dann wird mit eiskalter 2 N-Schwefelsäure und anschließend mit Eiswasser extrahiert, mit Natriumsulfat getrocknet und schließlich eingedampft. Ausbeute 38.3 g (93 % d.Th.), farbloses Öl.
c) 8-Brom-3-thia-octansäure-ethylester
Man rührt ein Gemisch aus 38.2 g (0.134 mol) 8-Methansulfonyloxy)-3-thia-octansäure-ethylester, 100 ml Aceton und 23.4 g (0.269 mol) Lithiumbromid drei Stdn. bei Raumtemperatur, saugt ab und dampft ein. Nach Aufnehmen in Methylenchlorid, Ausschütteln mit Wasser und Trocknen mit Natriumsulfat wird eingedampft. Nach Chromatographie an Kieselgel mit Ligroin + Ether (15 + 1 Vol)
Ausbeute 24.1 g (67 % d.Th.), farbloses Öl.
d) 8-(4-Chlor-benzolsulfonamido)-3-thia-octansäure-ethylester
Eine Lösung aus 28.5 g (0.149 mol) 4-Chlorbenzolsulfonamid, 80 ml Methanol und 13.8 ml einer 30proz. Natriummethylat-Lösung wird kurz gerührt, dann im Vakuum zur Trockne eingedampft. Man gibt nun 200 ml abs. DMF und 20.9 g (0.0743 mol) 8-Brom-3-thia-octansäure-ethylester zu und rührt 3 Stdn. bei 70-

80°C. Dann wird in Eiswasser eingerührt und mit Ether extrahiert. Die Etherphase wäscht man mit Wasser, trocknet mit Natriumsulfat und dampft ein. Die etherische Lösung des Eindampfrückstandes wird über eine kurze Kieselgelsäule filtriert, dann dampft man wiederum ein. Der ölige Rückstand kristallisiert beim Verreiben mit Ligroin. Nach Umkristallisieren aus wäßrigem Ethanol 19.0 g (67 % d.Th.) Produkt mit dem Schmp. 48-49°C.

e) 8-(4-Chlor-benzolsulfonamido)-3-thia-octansäure

Ein Gemisch aus 5.0 g (0.013 mol) des nach d) erhaltenen Esters, 20 ml Ethanol und 20 ml 2 N NaOH wird 2 Stdn. bei 50-60°C gerührt, dann destilliert man im Vakuum das Ethanol ab, versetzt mit etwas Wasser und extrahiert mit Ether. Die wäßrige Phase wird nun mit verd. HCl angesäuert und das ausfallende Produkt abgesaugt. Nach Umkristallisieren aus wäßrigem Ethanol Ausbeute 3.6 g (78 % d.Th.) mit dem Schmp. 93-94°C.

Beispiel 2

8-(4-Chlor-benzolsulfonamido)-3-thia-octansäure-3-oxid

a) 8-(4-Chlor-benzolsulfonamido)-3-thia-octansäure-ethylester-3-oxid

Man rührt ein Gemisch aus 5.9 g (13.2 mmol) 8-(4-Chlor-benzolsulfonamido)-3-thia-octansäure-ethylester, 200 ml Ethanol, 150 ml Wasser und 3.11 g (14.5 mmol) Natriumperiodat 8 Stdn. bei Raumtemperatur, destilliert dann im Vakuum das Ethanol ab und verdünnt den Eindampfrückstand mit etwas Wasser. Die wäßrige Phase wird mit Methylenchlorid extrahiert, der Extrakt wird mit Wasser gewaschen, dann getrocknet ($NaSO_4$) und eingedampft. Man verreibt den öligen Rückstand mit Ligroin, saugt das auskristallisierte Produkt ab und kristallisiert aus Ethanol um.

Ausbeute 4.0 g (77 % d.Th.), Schmp. 85-86°C.

b) Die freie Säure erhält man durch Verseifen des nach

a) erhaltenen Ethylesters in einer zu Beispiel 1 e analogen Verfahrensweise.

Ausbeute 76 % d.Th., Schmp. 91-92°C.

Beispiel 3

8-(4-Chlor-benzolsulfonamido)-3-thia-octansäure-3.3-dioxid

a) 8-(4-Chlor-benzolsulfonamido)-3-thia-octansäure-ethylester-3.3-dioxid

Man hält ein Gemisch aus 5.0 g (13.2 mmol) 8-(4-Chlor-benzolsulfonamido)-3-thia-octansäure-ethylester-3-oxid, 15 ml Ethanol, 8 ml Wasser und 5.65 g (26.4 mmol) Natriumperiodat 8 Stdn. auf Rückflußtemperatur und arbeitet in gleicher Weise auf wie in Beispiel 2 a) beschrieben.

Ausbeute 80 % d.Th., Schmp. 55-57°C (Ethanol).

b) Die freie Säure erhalt man durch Verseifen des nach

a) erhaltenen Ethylesters in einer zu Beispiel 1 e) analogen Verfahrensweise.

Ausbeute 73 % d.Th., Schmp. 193-194°C (aus 66proz. Ethanol)

Beispiel 4

9-(4-Chlor-benzolsulfonamido)-3-thia-nonansäure

wurde in Analogie zu Beispiel 1 über folgende Stufen dargestellt:

a) 9-Hydroxy-3-thia-nonansäure-ethylester aus 6-Chlor-hexanol und Thioglykolsäure-ethylester.
Ausbeute 93 % d.Th., farbloses Öl.

b) 9-(Methansulfonyloxy)-3-thia-nonansäure-ethylester
Rohausbeute 94 % d.Th., farbloses Öl.

c) 9-Brom-3-thia-nonansäure-ethylester
Ausbeute 78 % d.Th., farbloses Öl.

d) 9-(4-Chlor-benzolsulfonamido)-3-thia-nonansäure-ethylester
Ausbeute 72 % d.Th., farbloses Öl.

e) 9-(4-Chlor-benzolsulfonamido)-3-thia-nonansäure
Ausbeute 51 % d.Th., Schmp. 104-105°C (Heptan + Ethylacetat).

Beispiel 5

9-(4-Chlor-benzolsulfonamido)-3-thia-nonansäure-3-oxid

erhält man in zu Beispiel 2 analoger Weise durch Oxidation von 9-(4-Chlor-benzolsulfonamido)-3-thia-nonansäure-ethylester zu

a) 9-(4-Chlor-benzolsulfonamido)-3-thia-nonansäure-ethylester-3-oxid
Ausbeute 72 % d.Th., Schmp. 72-74°C (nach Chromatographie an Kieselgel mit Ethylacetat)
und Verseifen zur

b) Säure:
Ausbeute 62 % d.Th., Schmp. 105-106°C (aus 66proz. Ethanol).

Beispiel 6

9-(4-Chlor-benzolsulfonamido)-3-thia-nonansäure-3.3-dioxid

erhält man in zu Beispiel 3 analoger Weise durch Oxidation von 9-(4-Chlor-benzolsulfonamido)-3-thia-nonansäure-ethylester zu

a) 9-(4-Chlor-benzolsulfonamido)-3-thia-nonansäure-ethylester-3.3-dioxid
Ausbeute 92 % d.Th., farbloses Öl
und Verseifen zur

b) Säure:
Nach Chromatographie an Kieselgel mit einem Gemisch aus 40 Vol. Methylenchlorid, 8 Vol. Methanol und 1 Vol. Wasser: Ausbeute 51 % d.Th., Schmp. 109-110°C.

Beispiel 7

(Z)-9-(4-Chlor-benzolsulfonamido)-3-thia-7-en-nonansäure

a) (Z)-9-Tetrahydropyranyloxy-3-thia-7-en-nonansäure-ethylester
Zu einem Gemisch aus 70 ml Ethanol, 59 mmol Natriummethylat (in Form einer 30proz. methanolischen Lösung) und 7.13 g (59 mmol) Thioglykolsäureethylester tropft man bei Raumtemperatur eine Lösung aus 70 ml Ethanol und 13.0 g (59 mmol) (Z)-6-Tetrahydropyranyloxy-4-hexen-yl-chlorid und hält anschließend zwei Stdn. auf Rückflußtemperatur.
Danach wird im Vakuum Ethanol abdestilliert, der Rückstand mit Eiswasser versetzt und mehrmals ausgeethert. Man trocknet die etherische Lösung mit Natriumsulfat, dampft ein und fraktioniert im Vakuum. Ausbeute 13.4 g (75 % d.Th.), farbloses Öl, Sdp. bei 0.13 mbar: 165-167°C.

b) (Z)-9-Hydroxy-3-thia-7-en-nonansäure-ethylester
Ein Gemisch aus 50 ml Ethanol, 15 ml 2 N $H_2SO_4$ und 11.5 g (38 mmol) der nach a) erhaltenen Tetrahydropyranyloxy-verbindung wird 5 Stdn. bei 80°C gerührt. Dann destilliert man das Ethanol ab, gießt auf Eis und ethert aus. Die etherische Lösung wird mit verd. Natronlauge, dann mit Wasser extrahiert, über $MgSO_4$ getrocknet und eingedampft. Fraktionieren im Vakuum ergibt 3.5 g (42 % d.Th.) farbloses Öl, Sdp. bei 0.666 mbar: 135°C.

c) (Z)-9-Methansulfonyloxy-3-thia-7-en-nonansäure-ethylester
in Analogie zu Bsp. 1 b) aus der nach b) erhaltenen Hydroxyverbindung und Methansulfonylchlorid. Ausbeute 76 % d.Th., farbloses Öl.

d) (Z)-9-Brom-3-thia-7-en-nonansäure-ethylester
in Analogie zu Bsp. 1 c) aus der nach c) erhaltenen Methansulfonyloxy-verbindung und Lithiumbromid. Ausbeute 96 % d.Th., farbloses Öl.

e) (Z)-9-(4-Chlor-benzolsulfonamido)-3-thia-7-en-nonansäure-ethylester
in Analogie zu Bsp. 1 d) aus der nach d) erhaltenen Bromverbindung und 4-Chlor-benzolsulfonamid. Nach Chromatographie an Kieselgel mit einem Gemisch aus Ether (1 Vol.) und Cyclohexan (2 Vol.) Ausbeute 62 % d.Th., farbloses Öl.

f) (Z)-9-(4-Chlor-benzolsulfonamido)-3-thia-7-en-nonanäure
in Analogie zu Bsp. 1 e) durch Hydrolyse des Ethylesters. Ausbeute 81 % d.Th., farbloses Öl.

Beispiel 8

7-(4-Chlor-benzolsulfonamido)-2.2-dimethyl-5-thia-heptansäure

a) 7-Hydroxy-2.2-dimethyl-5-thia-heptansäure-ethylester
Ein Gemisch aus 5.6 g (25.1 mmol) 4-Brom-2.2-dimethyl-butansäure-ethylester, 50 ml Ethanol, 25.1 mmol Natriummethylat (in Form einer 30proz. methanolischen Lösung) und 2.0 g 2-Mercapto-ethanol wird drei Stdn. bei Raumtemperatur gerührt und danach im Vakuum eingedampft. Nun wird Wasser zugegeben und ausgeethert. Die etherische Lösung wird mit kalter 1 N NaOH extrahiert, mit Natriumsulfat getrocknet und eingedampft.
Ausbeute 5.0 g (90 % d.Th.), farbloses Öl.
b) 7-Brom-2.2-dimethyl-5-thia-heptansäure-ethylester
Durch Umsetzen der nach a) erhaltenen Hydroxyverbindung mit Methansulfonylchlorid in Analogie zu Bsp. 1 b) erhält man die entsprechende Methansulfonyloxyverbindung, welche in Analogie zu Bsp. 1 c) mit LiBr zur Bromverbindung umgesetzt wird.
Ausbeute roh: fast quantitativ, farbloses Öl.
c) 7-(4-Chlor-benzolsulfonamido)-2.2-dimethyl-5-thia-heptansäure-ethylester
in Analogie zu Bsp. 1 d) aus der nach b) erhaltenen Bromverbindung und 4-Chlor-benzolsulfonamid.
Ausbeute 68 % d.Th., farbloses Öl.
d) 7-(4-Chlor-benzolsulfonamido)-2.2-dimethyl-5-thia-heptansäure
durch Verseifen des Ethylesters in Analogie zu Bsp. 1 e).
Ausbeute 84 % d.Th., Schmp. 112-113°C.
In Analogie dazu lassen sich herstellen:

2. 8-(4-Chlor-benzolsulfonamido)-2.2-dimethyl-6-thia-octansäure

über folgende Stufen:
a) 8-Hydroxy-2.2-dimethyl-6-thia-octansäure-ethylester
aus 5-Brom-2.2-dimethyl-pentansäure-ethylester und 2-Mercapto-ethanol. Rohausbeute quantitativ, farbloses Öl.
b) 8-Brom-2.2-dimethyl-6-thia-octansäure-ethylester
aus 8-Hydroxy-2.2-dimethyl-6-thia-octansäure-ethylester und Methansulfonylchlorid und anschließende Umsetzung der entstandenen Methansulfonyloxyverbindung mit Lithiumbromid.
Ausbeute 62 % d.Th., farbloses Öl.
c) 8-(4-Chlor-benzolsulfonamido)-2.2-dimethyl-6-thia-octansäure-ethylester
aus 8-Brom- 2.2-dimethyl-6-thia-octansäure-ethylester und 4-Chlor-benzolsulfonamid.
Ausbeute 63 % d.Th., farbloses Öl.
d) 8-(4-Chlor-benzolsulfonamido)-2.2-dimethyl-6-thia-octansäure
durch Verseifen des Ethylesters.
Ausbeute 96 % d.Th., Schmp. 107-109°C.

3. 8-(4-Chlor-benzolsulfonamido)-3.3-dimethyl-6-thia-octansäure

über folgende Stufen:
a) 8-Hydroxy-3.3-dimethyl-6-thia-octansäure-ethylester
Ein Gemisch aus 50 ml DMSO und 42.2 mmol Natriumhydrid wird 45 min auf 80°C gehalten. Dann kühlt man auf Raumtemperatur ab, gibt 3.3 g (42.2 mmol) 2-Mercapto-ethanol und dann 10.0 g (42.2 mmol) 5-Brom-3.3-dimethyl-pentansäure-ethylester zu und rührt drei Stdn. bei Raumtemperatur. Dann gießt man in Wasser und ethert aus. Die Etherphase wird mit Natriumsulfat getrocknet, dann eingedampft. Nach Chromatographie an Kieselgel mit einem Gemisch aus 1 Vol. Ether und 1 Vol. Cyclohexan 6.8 g (70 % d.Th.), farbloses Öl.
b) 8-Brom-3.3-dimethyl-6-thia-octansäure-ethylester
aus 8-Hydroxy-3.3-dimethyl-6-thia-octansäure-ethylester und Methansulfonylchlorid und anschließende Umsetzung der entstandenen Methansulfonyloxyverbindung mit Lithiumbromid.
Ausbeute roh: quantitativ, farbloses Öl.
c) 8-(4-Chlor-benzolsulfonamido)-3.3-dimethyl-6-thia-octansäure-ethylester
aus 8-Brom-3.3-dimethyl-6-thia-octansäure-ethylester und 4-Chlor-benzolsulfonamid. Nach Chromatographie an Kieselgel mit einem Gemisch aus 2 Vol. Ether und 7 Vol. Ligroin Ausbeute 71 % d.Th., farbloses

Öl.
d) 8-(4-Chlor-benzolsulfonamido)-3.3-dimethyl-6-thia-octansäure
durch Verseifen des Ethylesters.
Ausbeute 92 % d.Th., farbloses Öl.

4. 9-(4-Chlor-benzolsulfonamido)-2,2-dimethyl-3-thia-nonansäure

über folgende Stufen:
a) 9-Hydroxy-2,2-dimethyl-3-thia-nonansäure-ethylester
in Analogie zu Bsp. 8a aus 2-Brom-2-methyl-propionsäure-ethylester und 6-Mercapto-hexanol.
Rohausbeute 74 % d.Th., farbloses Öl;
$n_D^{20}$ = 1.4890.
b) 9-Brom-2,2-dimethyl-3-thia-nonansäure-ethylester
aus 9-Hydroxy-2,2-dimethyl-3-thia-nonansäure-ethylester und Methansulfonylchlorid und anschließende Umsetzung der entstandenen Methansulfonyloxyverbindung mit Lithiumbromid.
Ausbeute nach Chromatografie an Kieselgel/Cyclohexan: 57 % d.Th., farbloses Öl;
$n_D^{20}$ = 1.4916.
c) 9-(4-Chlor-benzolsulfonamido)-2,2-dimethyl-3-thia-nonansäure-ethylester
aus 9-brom-2,2-dimethyl-3-thia-nonansäure-ethylester und 4-Chlor-benzolsulfonamid.
Nach Chromatografie an Kieselgel/Cyclohexan + Ether (1+1 Vol.)
Ausbeute 81 % d.Th., farbloses Öl.
$n_D^{20}$ = 1.5175.
d) Titelverbindung
durch Verseifen des Ethylesters.
Nach Chromatographie an Kieselgel/$CH_2Cl_2$
Ausbeute 67 % d.Th., Schmp. 93-94°C.

Beispiel 9

8-(4-Chlor-benzolsulfonamido)-2,2-dimethyl-3-thia-octansäure
a) 8-Hydroxy-2,2-dimethyl-3-thia-octansäure-ethylester
In Analogie zu Beispiel 8a aus 5-Mercaptopentanol und 2-Brom-2-methyl-propionsäure-ethylester.
Rohausbeute 75 % d.Th., farbloses Öl;
$n_D^{20}$ = 1.4800 (enthält etwas Methylester).
b) 8-Brom-2,2-dimethyl-3-thia-octansäure-ethylester
aus 8-Hydroxy-2,2-dimethyl-3-thia-octansäure-ethylester und Methansulfonylchlorid und anschließende Umsetzung der entstandenen Methansulfonyloxyverbindung mit Lithiumbromid.
Ausbeute 86 % d.Th., farbloses Öl,
$n_D^{20}$ = 1.4936 (enthalt etwas Methylester).
c) 8-(4-Chlor-benzolsulfonamido)-2,2-dimethyl-3-thia-octansäure-ethylester
aus 8-brom-2,2-dimethyl-3-thia-octansäure-ethylester und 4-Chlor-benzolsulfonamid.
Nach Chromatografie an Kieselgel/Cyclohexan + Ether (1+1 Vol.)
Ausbeute 79 % d.Th., farbloses Öl.
$n_D^{20}$ = 1.5331 (enthält etwas Methylester).
d) 8-(4-Chlor-benzolsulfonamido)-2,2-dimethyl-3-thia-octansäure
durch Verseifen des Ethylesters.
Nach Chromatografie an Kieselgel/$CH_2Cl_2$
Ausbeute 64 % d.Th., Schmp. 98°C (Toluol).
In analoger Weise erhält man

2. 8-(4-Methoxy-benzolsulfonamido)-2,2-dimethyl-3-thia-octansäure

durch Umsetzen von 8-Brom-2,2-dimethyl-3-thia-octansäure-ethylester mit 4-Methoxy-benzolsulfonamid zu
a) 8-(4-Methoxy-benzolsulfonamido)-2,2-dimethyl-3-thia-octansäure-ethylester
Nach Chromatografie an Kieselgel/Cyclohexan + Ether (2+1 Vol.)
Ausbeute 69 % d.Th., farbloses Öl.
$n_D^{20}$ = 1.5280
und nachfolgende Verseifung dieses Esters zur

b) Säure
Ausbeute 90 % d.Th., Schmp. 57-59°C.

3. 8-(3-Trifluormethyl-benzolsulfonamido)-2,2-dimethyl-3-thia-octansäure

durch Umsetzen von 8-Brom-2,2-dimethyl-3-thia-octansäure-ethylester mit 3-Trifluormethyl-benzolsulfonamid zu

a) 8-(3-Trifluormethyl-benzolsulfonamido)-2,2-dimethyl-3-thia-octansäure-ethylester
Nach Chromatografie an Kieselgel/Cyclohexan + Ether (2+1 Vol.)
Ausbeute 75 % d.Th., farbloses Öl.
$n_D^{20}$ = 1.4930.
und nachfolgende Verseifung dieses Esters zur
b) Säure
Ausbeute 88 % d.Th., Schmp. 93-94°C.

Beispiel 10

9-(4-Chlor-benzolsulfonamido)-3,3-dimethyl-4-thia-nonansäure

a) 9-Methansulfonyloxy-3,3-dimethyl-4-thia-nonansäure-ethylester
Zu einem Gemisch aus 4.0 g (16.1 mmol) 9-Hydroxy-3,3-dimethyl-4-thia-nonansäure-ethylester (dargestellt aus β,β-Dimethyl-acrylsäure-ethylester und 5-Mercaptopentanol in Analogie zu Vovrek et al., J.Med.Chem. 15 (1972), S. 125), 3.26 g (32.2 mmol) abs. Triethylamin und 25 ml Methylenchlorid tropft man bei 0°C eine Lösung aus 1.85 g (16.1 mmol) Methansulfonylchlorid und 20 ml Methylenchlorid, läßt eine Stunde bei Raumtemperatur nachrühren und wäscht anschließend mit eiskalter 1 N-HCl und Eiswasser. Dann wird mit $MgSO_4$ getrocknet und i.Vak. eingedampft. Ausbeute 5.1 g (96 % d.Th.), farbloses Öl.

b) 9-Brom-3,3-dimethyl-4-thia-nonansäure-ethylester
Ein Gemisch aus 5.0 g (15.3 mmol) 9-Methansulfonyloxy-3,3-dimethyl-4-thia-nonansäure-ethylester, 25 ml Aceton und 1.5 g (17.3 mmol) LiBr wird 8 Stunden bei Raumtemperatur gerührt, dann i.Vak. eingedampft. Zum Rückstand gibt man Ether und Wasser, schüttelt durch und trennt die Etherphase ab. Die wäßrige Phase wird nochmals mit Ether extrahiert. Man trocknet die vereinigten Etherphasen ($Na_2SO_4$) und dampft ein. Zur Reinigung wird an einer Kieselgelsäule mit Cyclohexan + Ether (2+1 Vol.) chromatografiert. Ausbeute 3.6 g (76 % d.Th.), farbloses Öl.

c) 9-(4-Chlor-benzolsulfonamido)-3,3-dimethyl-4-thia-nonansäure-ethylester
durch Umsetzen des nach b) erhaltenen Brom-esters mit 4-Chlor-benzolsulfonamid in Analogie zu Beispiel 1d).
Nach Chromatografie an einer Kieselgelsäule mit Cyclohexan + Ether (2+1 Vol.)
Ausbeute 57 % d.Th., farbloses Öl.
$n_D^{20}$ = 1.5290.

d) Titelverbindung
durch Verseifen des nach c) erhaltenen Ethylesters in einer zu Beispiel 1e analogen Verfahrensweise.
Ausbeute 71 % d.Th., Schmp. 46-48°C.

Beispiel 11

9-(4-Chlor-benzolsulfonamido)-2,2-dimethyl-6-thia-nonansäure

a) 9-(4-Chlor-benzolsulfonamido)-2,2-dimethyl-6-thia-nonansäure-ethylester
erhält man durch Umsetzen von 3-(4-Chlor-benzolsulfonamido)propylmercaptan mit 5-Brom-2,2-dimethyl-pentansäure-ethylester in Analogie zu Beispiel 8a.
Nach Chromatografie an Kieselgel/Cyclohexan + Ethanol (100 + 1.2 Vol.) Ausbeute 61 % d.Th., farbloses Öl.

b) Die Säure
erhält man durch Verseifen des Ethylesters analog Beispiel 1e.
Ausbeute 91 % d.Th., farbloses Öl.

In analoger Weise wurde dargestellt:

2. 9-(4-Chlor-benzolsulfonamido)-3,3-dimethyl-6-thia-nonansäure

a) 9-(4-Chlor-benzolsulfonamido)-3,3-dimethyl-6-thia-nonansäure-ethylester
erhält man durch Umsetzen von 3-(4-Chlor-benzolsulfonamido)propylmercaptan mit 5-Brom-3,3-dimethyl-pentansäure-ethylester in Analogie zu Beispiel 8a.
Nach Chromatografie an Kieselgel/Cyclohexan + Ether (8+2 Vol.)
Ausbeute 66 % d.Th., farbloses Öl.
b) Die Säure
erhält man durch Verseifen des Ethylesters analog Beispiel 1e.
Nach Chromatografie an Kieselgel/Cyclohexan + Ethanol (8+2 Vol.)
Ausbeute 71 % d.Th.; farbloses Öl.

Beispiel 12

9-(4-Chlor-benzolsulfonamido)-3-oxa-nonansäure

a) Zu einer Suspension von 0,4 g Kupferpulver in einer leicht siedenden Lösung von 5,0 g 6-Bromhexanol in 15 ml Essigester wird unter heftigem Rühren 15 Minutenlang eine kalte Lösung von 2,5 g Diazoessigsäureethylester in 5 ml Essigester zugetropft. Danach wird 4 Std. unter Rückfluß gerührt. Nach dem Abkühlen wird die Lösung filtriert und das Lösungsmittel im Vakuum abdestilliert, wobei 4,7 g eines öligen Rückstandes erhalten wird. In dem Rückstand werden 35 ml Essigester 5,0 g 6-Bromhexanol und zusätzlich 0,4 g Kupferpulver zugegeben und die oben beschriebene Reaktion wiederholt, wobei eine Lösung von 3,4 g Diazoessigsäureethylester in 15 ml Essigester zugegeben wird.
Nach Abdestillieren des Lösungsmittels wird ein bernsteinfarbenes Öl (9,2 g) erhalten, das über eine Silicagel-Säule mit Chloroform gereinigt wird. Es werden 5,2 g 6-Brcmhexyloxyessigsäureethylester als farbloses Öl erhalten.
b) Eine Mischung von 2,0 g 6-Bromhexyloxyessigsäureethylester, 4,0 g 4-Chlor-benzolsulfonamid und 8,7 g wasserfreies Kaliumcarbonat in 25 ml trockenem Dimethylformamid werden 4 Stundenlang bei 110° -120°C gerührt. Die Mischung wird im Vakuum soweit eingeengt, bis ein schmieriger Brei entsteht, zu dem Wasser und Dichlormethan gegeben wird. Die organische Phase wird mit Wasser gewaschen, getrocknet und eingedampft, wobei 4,4 g eines orangenfarbigen Öls erhalten wird. Die Reinigung erfolgt mit einer Chlorform/Essigester-Mischung auf einer Silicagel-Säule. Es werden 800 mg 9-(4-Chlor-benzolsulfon-amido)-3-oxa-nonansäureethylester als farbloses öl erhalten.
c) 700 mg 9-(4-Chlor-benzolsulfonamido)-3-oxa-nonansäureethylester werden mit einer warmen Mischung aus verdünnter Natronlauge und Ethanol hydrolysiert. Die Mischung wird in Vakuum auf 20 % des Volumens eingeengt, mit Ether extrahiert und mit verdünnter Schwefelsäure auf ca. pH 2 angesauert. Die Mischung wird mit Dichlormethan extrahiert und der getrocknete Extrakt eingedampft, wobei ein farbloses Öl entsteht, das bei längerem Stehen fest wird. Nach Umkristallisation mit Dichlormethan/Ether erhält man 300 mg 9-(4-Chlor-benzolsulfonamido)-3-oxa-nonansäure.
Fp: 91 - 92°C.

**Patentansprüche**

**1.** Verbindungen der Formel I

$$\begin{array}{l} R_1\text{-}SO_2\text{-}\underset{\displaystyle R_2}{\underset{|}{N}}\text{-}A\text{-}Q\text{-}B\text{-}Y \end{array} \qquad (I)$$

in der

R₁ eine niedere Alkyl- oder Alkenylgruppe mit 1-6 C-Atomen, einen Cycloalkylrest mit 3-7 C-Atomen, einen Aralkyl-, Aralkenyl- oder Arylrest, wobei der jeweilige Arylrest ein- oder mehrfach durch Halogen, $C_1$- $C_6$-Alkyl, $C_1$-$C_6$-Alkoxy, Hydroxyl, Trifluormethyl, Cyan, Nitro, Amino, $C_1$-$C_6$-Alkylamino, $C_2$-$C_{12}$-Dialkylamino, $C_1$-$C_6$-Acylamino, $C_1$-$C_{16}$-Acyl,

$C_1$-$C_6$-Alkylsulfenyl, -sulfinyl und -sulfonyl oder durch Azid substituiert sein kann,

$R_2$ ein Wasserstoffatom, eine $C_1$-$C_6$-Alkyl-, eine Aralkyl-, eine Aralkenyl- oder eine Acylgruppe,

A und B eine gesättigte oder ungesättigte Alkylenkette mit 1-10 Kohlenstoffatomen, die ein- oder mehrfach duch $C_1$-$C_3$-Alkylgruppen substituiert sein kann, wobei die Summe der Kohlenstoffatome der Ketten A plus B wenigstens 4, maximal jedoch 11 beträgt,

Q Sauerstoff, Schwefel, eine Sulfonyl- oder eine Sulfinylgruppe, oder eine Aminogruppe

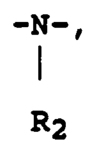

wobei für $R_2$ die obige Definition gilt,

Y eine freie Carbonsäuregruppe, ein Carbonsäureester, ein Carbonsäureamid oder Hydroxymethyl oder Tetrazolyl

bedeuten,

deren pharmakologisch verträgliche Salze und optische sowie E-Z-Isomere bzw. deren Gemische.

**2.** Verbindungen gemäß Anspruch 1, in denen $R_1$ 4-Chlorphenyl, 4-Methoxyphenyl oder 4-Trifluormethylphenyl bedeutet.

**3.** Verbindungen gemäß Anspruch 1 oder 2, in denen A die Gruppe $-(CH_2)_n$, in der n die Zahlen 1 bis 6 bedeuten, oder die Gruppe $-(CH_2)_m-CH=CH-(CH_2)_n$, in der m die Zahlen 1 oder 2 und n die Zahlen 2, 3, 4 oder 5 bedeuten, darstellt.

**4.** Verbindungen gemäß Anspruch 1, 2 oder 3, in denen B die Gruppe $-(CH_2)_p-$, in der p die Zahlen 1 bis 7 bedeutet, die Gruppe $-(CH_2)_q-C(CH_3)2-$, in der q die Zahlen 0 bis 6, oder die Gruppe $-(CH_2)_r-C(CH_3)_2-CH_2-$, in der r die Zahlen 0 bis 5 bedeutet, darstellen.

**5.** Verfahren zur Herstellung von Verbindungen der Formel I

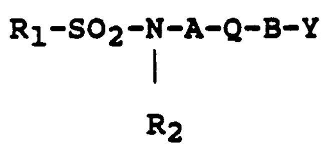

(I)

in der

$R_1$ eine niedere Alkyl- oder Alkenylgruppe mit 1-6 C- Atomen, einen Cycloalkylrest mit 3-7 C-Atomen, einen Aralkyl-, Aralkenyl- oder Arylrest, wobei der jeweilige Arylrest ein- oder mehrfach durch Halogen, $C_1$-$C_6$-Alkyl, $C_1$-$C_6$-Alkoxy, Hydroxyl, Trifluormethyl, Cyan, Nitro, Amino, $C_1$-$C_6$-Alkylamino, $C_2$-$C_{12}$-Dialkylamino, $C_1$-$C_6$-Acylamino, $C_1$-$C_{16}$-Acyö, $C_1$-$C_6$-Alkylsulfenyl, -sulfinyl und -sulfonyl oder durch Azid substituiert sein kann,

$R_2$ ein Wasserstoffatom, eine $C_1$-$C_6$-Alkyl-, eine Aralkyl-, eine Aralkenyl- oder eine Acylgruppe,

A und B eine gesättigte oder ungesättigte Alkylenkette mit 1-10 Kohlenstoffatomen, die ein- oder mehrfach durch $C_1$-$C_3$-Alkylgruppen substituiert sein kann, wobei die Summe der Kohlenstoffatome der Ketten A plus B wenigstens 4, maximal jedoch 11 beträgt,

Q Sauerstoff, Schwefel, eine Sulfonyl- oder eine Sulfinyl-gruppe, jeder eine Aminogruppe

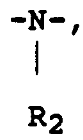

wobei für $R_2$ die obige Definition gilt,

Y eine freie Carbonsäuregruppe, ein Carbonsäureester, ein Carbonsäureamid oder Hydroxymethyl oder Tetrazolyl

bedeuten,

deren pharmakologisch verträgliche Salze und optische sowie E-Z-Isomere bzw. deren Gemische, dadurch gekennzeichnet, daß man in an sich bekannter Weise

a) eine Verbindung der Formel V

X-A-Q-B-Y (V)

in der

A, B und Y die angegebene Bedeutung haben,

X einen reaktiven Rest und

Q Sauerstoff, Schwefel oder eine NH-Gruppe darstellen,

mit einem Sulfonamid der Formel VI

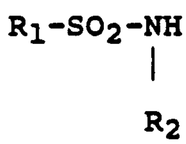

(VI)

in der

$R_1$ und $R_2$ die angegebene Bedeutung haben,

umsetzt,

anschließend gewünschtenfalls den Schwefel zu Sulfinyl- oder Sulfonyl-Gruppen oxidiert sowie die NH-Gruppe in üblicher Weise mit $R_2$ substituiert

oder

b) für den Fall, daß Q eine N-$R_2$-Gruppe darstellte, eine Verbindung der Formel XIV

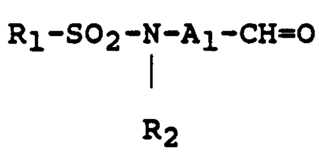

(XIV)

in der

$R_1$ und $R_2$ die angegebene Bedeutung haben und $A_1$ ein um 1 C-Atom verkürzter Rest A darstellen,

mit einer Verbindung der Formel X

$H_2N$-B-Y (X)

in der

B und Y die oben angegebene Bedeutung haben, umsetzt bzw.

eine Verbindung der Formel XV

$$R_1-SO_2-\underset{\displaystyle R_2}{\underset{|}{N}}-A-NH_2 \qquad (XV)$$

in der
$R_1$, $R_2$ und A die aangegebene Bedeutung haben, mit einer Verbindung der Formel XII

$O=CH\text{-}B^1\text{-}Y$  (XII)

in der
Y die angegebene Bedeutung hat und $B^1$ ein um 1 C-Atom verkürzter Rest B darstellt, umsetzt und die erhaltenen Schiffschen Basen hydriert und anschließend gegebenenfalls die Gruppe N-H in üblicher Weise mit $R^2$ substituiert,
und gewünschtenfalls die erhaltenen Isomerengemische in üblicher Weise auftrennt sowie die erhaltenen Verbindungen in pharmakologisch verträgliche Salze überführt.

6. Arzneimittel, enthaltend eine Verbindung gemäß Anspruch 1, 2, 3 oder 4 sowie übliche Träger- und Hilfsstoffe.

7. Arzneimittel, enthaltend eine Verbindung gemäß Anspruch 1, 2, 3 oder 4 zur Behandlung von Stoffwechselerkrankungen.

8. Verwendung von Verbindungen gemäß Anspruch 1, 2, 3 oder 4 zur Herstellung von Arzneimitteln zur Behandlung von Durchblutungsstörungen.

## Claims

1. Compounds of the formula I

$$R_1-SO_2-\underset{\displaystyle R_2}{\underset{|}{N}}-A-Q-B-Y \qquad (I)$$

in which $R_1$ signifies a lower alkyl or alkenyl group with 1 - 6 C-atoms, a cycloalkyl radical with 3 - 7 C-atoms, an aralkyl, aralkenyl or aryl radical, whereby the aryl radical in question can be substituted one or more times by halogen, $C_1$-$C_6$-alkyl, $C_1$-$C_6$-alkoxy, hydroxyl, trifluoromethyl, cyano, nitro, amino, $C_1$-$C_6$-alkylamino, $C_2$-$C_{12}$-dialkylamino, $C_1$-$C_6$-acylamino, $C_1$-$C_{16}$-acyl, $C_1$-$C_6$-alkylsulphenyl, -sulphinyl and -sulphonyl or by azido, $R_2$ a hydrogen atom, a $C_1$-$C_6$-alkyl, an aralkyl, an aralkenyl or an acyl group, A and B a saturated or unsaturated alkylene chain with 1 - 10 C-atoms which can be substituted one or more times by $C_1$-$C_3$-alkyl groups, whereby the sum of the carbon atoms of the chains A plus B amounts to at least 4 but maximum 11, Q oxygen, sulphur, a sulphonyl or a sulphinyl group or an amino group

$$-\underset{\displaystyle R_2}{\underset{|}{N}}- \; ,$$

$-N(R_2)-$, whereby the above definition applies for $R_2$, Y a free carboxylic acid group, a carboxylic acid ester, a carboxylic acid amide or hydroxymethyl or tetrazolyl; their pharmacologically acceptable salts and optical as well as E-Z isomers or their mixtures.

**2.** Compounds according to claim 1, in which $R_1$ signifies 4-chlorophenyl, 4-methoxyphenyl or 4-trifluoromethylphenyl.

**3.** Compounds according to claim 1 or 2, in which A represents the group $-(CH_2)_n-$, in which n signifies the numbers 1 to 6, or the group $-(CH_2)_m-CH=CH(CH_2)_n-$, in which m signifies the numbers 1 or 2 and n the numbers 2, 3, 4 or 5.

**4.** Compounds according to claim 1, 2 or 3, in which B represents the group $-(CH_2)_p-$, in which p signifies the numbers 1 to 7, the group $-(CH_2)q-C(CH_3)_2-$, in which q signifies the numbers 0 to 6, or the group $-(CH_2)_r-C(CH_3)_2-CH_2-$, in which r signifies the numbers 0 to 5.

**5.** Process for the preparation of compounds of the formula I

$$R_1-SO_2-\underset{\substack{| \\ R_2}}{N}-A-Q-B-Y \qquad (I)$$

in which $R_1$ signifies a lower alkyl or alkenyl group with 1 - 6 C-atoms, a cycloalkyl radical with 3 - 7 C-atoms, an aralkyl, aralkenyl or aryl radical, whereby the aryl radical in question can be substituted one or more times by halogen, $C_1$-$C_6$-alkyl, $C_1$-$C_6$-alkoxy, hydroxyl, trifluoromethyl, cyano, nitro, amino, $C_1$-$C_6$-alkylamino, $C_2$-$C_{12}$-dialkylamino, $C_1$-$C_6$-acylamino, $C_1$-$C_6$-alkylamino, $C_2$-$C_{12}$-dialkylamino, $C_1$-$C_6$-acylamino, $C_1$-$C_{16}$-acyl, $C_1$-$C_6$-alkylsulphenyl, -sulphinyl or -sulphonyl or by azido, $R_2$ a hydrogen atom, a $C_1$-$C_6$-alkyl, an aralkyl, an aralkenyl or acyl group, A and B a saturated or unsaturated alkylene chain with 1 - 10 carbon atoms which can be substituted one or more times by $C_1$-$C_3$-alkyl groups, whereby the sum of carbon atoms of the chains A plus B amounts to at least 4 but maximum 11, Q oxygen, sulphur, a sulphonyl or a sulphinyl group or an amino group

$$-\underset{\substack{| \\ R_2}}{N}- \ ,$$

whereby the above definition applies for $R_2$, Y a free carboxylic acid group, a carboxylic acid ester, a carboxylic acid amide or hydroxymethyl or tetrazolyl; of their pharmacologically acceptable salts and optical as well as E-Z isomers or of their mixtures, characterised in that, in per se known manner, one

a) reacts a compound of the formula V

X-A-Q-B-Y (V)

in which A, B and Y have the given meaning, X represents a reactive residue and Q oxygen, sulphur or an -NH-group, with a sulphonamide of the formula VI

$$R_1-SO_2-\underset{\substack{| \\ R_2}}{NH} \qquad (VI)$$

in which $R_1$ and $R_2$ have the given meaning, and subsequently, if desired, oxidises the sulphur to sulphinyl or sulphonyl groups, as well as substitutes the -NH- group in the usual way with $R_2$; or

b) for the case that Q represents an $-N(R_2)-$ group, reacts a compound of the formula XIV

$$R_1-SO_2-\underset{\substack{| \\ R_2}}{N}-A_1-CH=O \qquad (XIV)$$

in which $R_1$ and $R_2$ have the given meaning and $A_1$ represents a radical A shortened by 1 C-atom,

with a compound of the formula X

$H_2$-N-B-Y (X)

in which B and Y have the given meaning; or reacts a compound of the formula XV

$$R_1-SO_2-\underset{\displaystyle R_2}{\underset{|}{N}}-A-NH_2 \qquad (XV)$$

in which $R_1$, $R_2$ and A have the given meaning, with a compound of the formula XII

O = CH-$B^1$-Y (XII)

in which Y has the given meaning and $B^1$ represents a radical B shortened by 1 C-atom, and hydrogenates the Schiff bases obtained and subsequently possibly substitutes the group NH with $R_2$ in the usual way and, if desired, separates the isomeric mixture obtained in the usual way, as well as converts the compounds obtained into pharmacologically acceptable salts.

**6.** Medicaments containing a compound according to claim 1, 2, 3 or 4, as well as usual carrier and auxiliary materials.

**7.** Medicaments containing a compound according to claim 1, 2, 3 or 4 for the treatment of metabolic diseases.

**8.** Use of compounds according to claim 1, 2, 3 or 4 for the production of medicaments for the treatment of circulatory disturbances.

## Revendications

**1.** Composés de formule I

$$R_1-SO_2-\underset{\displaystyle R_2}{\underset{|}{N}}-A-Q-B-Y \qquad (I)$$

dans laquelle

- $R_1$ représente un groupe alkyle ou alcényle inférieur comportant 1-6 atomes de C, un groupe cycloalkyle comportant 3-7 atomes de C, un groupe aralkyle, aralcényle ou aryle, chaque groupe aryle pouvant être substitué une ou plusieurs fois par un atome d'halogène, un groupe alkyle en $C_1$-$C_6$,alcoxy en $C_1$-$C_6$, hydroxy, trifluorométhyle, cyano, nitro, amino, alkyl($C_1$-$C_6$)-amino, dialkyl($C_2$-$C_{12}$)-amino, acyl($C_1$-$C_6$)-amino, acyle en $C_1$-$C_{16}$, alkyl($C_1$-$C_6$)-sulfényle, sulfinyle et sulfonyle, ou par un groupe azide,
- $R_2$ représente un atome d'hydrogène, un groupe alkyle en $C_1$-$C_6$, un groupe aralkyle, aralcényle, ou un groupe acyle,
- A et B représentent une chaîne alkylène saturée ou insaturée comportant 1-10 atomes de carbone, qui peut être substituée une ou plusieurs fois par des groupes alkyle en $C_1$-$C_3$, la somme des atomes de carbone de la chaîne A plus ceux de la chaîne B étant d'au moins 4, et d'au plus 11,
- Q représente un atome d'oxygène, de soufre, un groupe sulfonyle ou un groupe sulfinyle, ou un groupe amino

$$\begin{array}{c} -N-, \\ | \\ R_2 \end{array}$$

dans lequel $R_2$ répond a' la définition ci-dessus,

Y — représente un groupe acide carboxylique libre, un ester carboxylique, un amide carboxylique ou un groupe hydroxyméthyle ou tétrazolyle,

leurs sels pharmacologiquement acceptables et leurs isomères optiques ainsi que leurs isomères E-Z, et leurs mélanges.

**2.** Composés selon la revendication 1, dans lesquels $R_1$ représente un groupe 4-chlorophényle, 4-méthoxyphényle ou 4-trifluorométhylphényle.

**3.** Composés selon la revendication 1 ou 2, dans lesquels A représente un groupe $-(CH_2)_n-$, dans lequel n représente un nombre de 1 à 6, ou le groupe $-(CH_2)_m-CH=CH-(CH_2)_n-$, dans lequel m vaut 1 ou 2 et n représente le nombre 2, 3, 4 ou 5.

**4.** Composés selon la revendication 1, 2 ou 3, dans lesquels B représente le groupe $-(CH_2)_p-$ dans lequel p représente un nombre de 1 à 7, le groupe $-(CH_2)_q-C(CH_3)_2-$ dans lequel Q représente un nombre de 0 à 6, ou le groupe $-(CH_2)_r-C(CH_3)_2-CH_2-$, dans lequel r représente un nombre de 0 à 5.

**5.** Procédé de préparation des composés de formule I

$$\begin{array}{c} R_1-SO_2-N-A-Q-B-Y \\ | \\ R_2 \end{array} \qquad (I)$$

dans laquelle

$R_1$ — représente un groupe alkyle ou alcényle inférieur comportant 1-6 atomes de C, un groupe cycloalkyle comportant 3-7 atomes de C, un groupe aralkyle, aralcényle ou aryle, chaque groupe aryle pouvant être substitué une ou plusieurs fois par un atome d'halogène, un groupe alkyle en $C_1$-$C_6$,alcoxy en $C_1$-$C_6$, hydroxy, trifluorométhyle, cyano, nitro, amino, alkyl($C_1$-$C_6$)-amino, dialkyl($C_2$-$C_{12}$)-amino, acyl($C_1$-$C_6$)-amino, acyle en $C_1$-$C_{16}$, alkyl($C_1$-$C_6$)-sulfényle, sulfinyle et sulfonyle, ou par un groupe azide,

$R_2$ — représente un atome d'hydrogène, un groupe alkyle en $C_1$-$C_6$, un groupe aralkyle, aralcényle, ou un groupe acyle,

A et B — représentent une chaîne alkylène saturée ou insaturée comportant 1-10 atomes de carbone, qui peut être substituée une ou plusieurs fois par des groupes alkyle en $C_1$-$C_3$, la somme des atomes de carbone de la chaîne A plus ceux de la chaîne B étant d'au moins 4, et d'au plus 11,

Q — représente un atome d'oxygène, de soufre, un groupe sulfonyle ou un groupe sulfinyle, ou un groupe amino

$$\begin{array}{c} -N-, \\ R_2 \end{array}$$

dans lequel $R_2$ répond à la définition ci-dessus,

Y — représente un groupe acide carboxylique libre, un ester carboxylique, un amide carboxylique ou un groupe hydroxyméthyle ou tétrazolyle,

leurs sels pharmacologiquement acceptables et leurs isomères optiques ainsi que leurs isomères E-Z, et leurs mélanges, caractérisé en ce que de manière connue en soi, on fait réagir

a) un composé de formule V

X-A-Q-B-Y (V)

dans laquelle
A, B et Y ont la signification mentionnée,
X représente un groupe réactif et
Q représente un atome d'oxygène, de soufre ou un groupe NH,
avec un sulfamide de formule VI

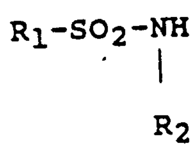

(VI)

dans laquelle
$R_1$ et $R_2$ ont la signification mentionnée,
et ensuite, on oxyde éventuellement le soufre en groupe sulfinyle ou sulfonyle, et l'on substitue le groupe NH de manière habituelle par $R_2$
ou
b) dans le cas où Q représente un groupe N-$R_2$, on fait réagir un composé de formule XIV

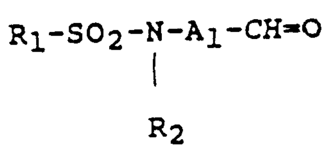

(XIV)

dans laquelle $R_1$ et $R_2$ ont la signification mentionnée et $A_1$ représente un groupe A avec un atome de C en moins,
avec un composé de formule X

$H_2N$-B-Y (X)

dans laquelle
B et Y ont la signification mentionnée,
ou l'on fait réagir un composé de formule XV

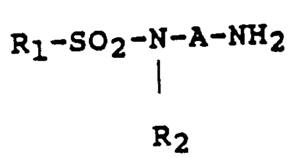

(XV)

dans laquelle
$R_1$, $R_2$ et A ont la signification mentionnée,
avec un composé de formule XII

O = CH-$B^1$-Y (XII)

dans laquelle
Y a la signification mentionnée et B représente un groupe $B^1$ avec un atome de C en moins,
et l'on hydrogène les bases de Schiff obtenues et ensuite, on substitue éventuellement le groupe N-H de manière usuelle par $R_2$, et éventuellement, on sépare les mélanges d'isomères obtenus de la

manière habituelle, et l'on transforme les composés obtenus en leurs sels pharmacologiquement acceptables.

**6.** Médicaments contenant un composé selon la revendication 1, 2, 3 ou 4, ainsi que des véhicules et adjuvants usuels.

**7.** Médicaments contenant un composé selon la revendication 1, 2, 3 ou 4, destinés au traitement des troubles du métabolisme.

**8.** Utilisation des composés selon la revendication 1, 2, 3 ou 4 pour la préparation de médicaments destinés au traitement de troubles circulatoires.